# EUROPÄISCHE PATENTANMELDUNG

(11) **EP 4 599 924 A1**
(43) Veröffentlichungstag der Anmeldung: **13.08.2025**
(21) Anmeldenummer: 25156223.7
(22) Anmeldetag: 06.02.2025
(51) Int. Cl.: B01D 61/12, B01D 65/02, A61M 1/16, C02F 1/44

(54) **SYSTEM UND VERFAHREN ZUR THERMISCHEN DESINFEKTION EINER UMKEHROSMOSEANLAGE**

(30) Priorität: 08.02.2024 DE 102024103544
(71) Anmelder: B. Braun Avitum AG, 34212 Melsungen (DE)
(72) Erfinder: LIEB, Nino, 34212 Melsungen (DE); ODERNHEIMER, Philipp, 34212 Melsungen (DE); GIESA, Jonas, 34121 Kassel (DE)
(74) Vertreter: Tergau & Walkenhorst Intellectual Property GmbH

(57) **Zusammenfassung**

System (120) aus Umkehrosmoseanlage (100) und Heißreinigungsanlage (103) zur thermischen Desinfektion der Umkehrosmoseanlage (100), die Umkehrosmoseanlage (100) umfassend wenigstens eine Druckerhöhungspumpe (112) und ein Membranmodul (114),
wobei die Umkehrosmoseanlage (100) über eine Zulaufleitung (101) hydraulisch mit der Heißreinigungsanlage (103) verbunden ist, und wobei eine Rücklaufleitung (106), in welche ein Magnetventil (105) geschaltet ist, von der Heißreinigungsanlage (103) zur Umkehrosmoseanlage (100) führt, und wobei die Zulaufleitung (101) hydraulisch mit der Rücklaufleitung (106) durch eine Bypassleitung (102) verbunden ist, in welche ein Bypassventil (104) geschaltet ist, welches einen Fluss durch die Bypassleitung (102) vollständig freigibt, wenn der Druck in der Zulaufleitung (101) größer ist als ein Haltedruck des Bypassventils (104),
wobei die Umkehrosmoseanlage (100) eine Steuerungseinheit (107) aufweist, welche folgende Schritte durchführt:
a) Öffnen des Magnetventils (105) und Leiten von erhitztem Wasser aus der Heißreinigungsanlage (103) durch die Rücklaufleitung (106) in die Um-kehrosmoseanlage (100);
b) Überprüfen des Desinfektionszustands der Umkehrosmoseanlage (100);
c) Sofern ein in der Steuerungseinheit (107) hinterlegtes Desinfektionsziel der Umkehrosmoseanlage (100) erreicht ist, Schließen des Magnetventils (105).

## Beschreibung

Die Erfindung betrifft ein System und ein Verfahren zur thermischen Desinfektion einer Umkehrosmoseanlage.

Umkehrosmoseanlagen müssen bedarfsweise oder in regelmäßigen Abständen desinfiziert werden. Eine derartige Desinfektion kann thermisch erfolgen, in dem die Umkehrosmoseanlage mit einer Heißreinigungsanlage hydraulisch verbunden wird. Die Heißreinigungsanlage fungiert dabei nach der Art eines Durchlauferhitzers und erhitzt das Wasser, welches ihr aus der Umkehrosmoseanlage zugeführt wird, und fördert dieses erhitzte Wasser in die Umkehrosmoseanlage zurück.

Bei bekannten Verfahren zur Desinfektion einer Umkehrosmoseanlage werden die Umkehrosmoseanlage und die Heißreinigungsanlage miteinander verbunden. Die beiden Anlagen weisen jeweils eine Steuereinheit auf, welche rudimentäre Kommandos (Heißreinigungsmodus aktiv, Heißreinigungsanlage benötigt Permeat, d.h. gefiltertes Wasser, etc.) miteinander austauschen. Da beide Anlagen jeweils eine Steuerungseinheit aufweisen, werden diese Kommandos bzw. Aufforderungen jeweils unabhängig ausgewertet und entsprechende Aktionen durchgeführt.

Der Vorgang und die Erfolgskontrolle der thermischen Desinfektion wird ebenfalls unabhängig von beiden Anlagen, respektive deren Steuerungseinheiten, durchgeführt. Dies bedeutet, dass das Desinfektionsziel bzw. das Erfolgskriterium einer Umkehrosmoseanlage ein anderes sein kann als das der Heißreinigungsanlage.

In dem oben dargestellten Beispiel kann es sich im Sinne des Desinfektionszieles um eine Kombination aus Zeit und einer Mindesttemperatur handeln. Das bedeutet allerdings, dass die Heißreinigungsanlage nicht oder nur unter hohem Aufwand mit Umkehrosmoseanlagen kompatibel ist, welche einen A₀-Wert als Erfolgskontrolle verwenden. Außerdem muss immer eine Form von Datenübertragung zwischen der Umkehrosmoseanlage und dem Heißreinigungsgerät vorhanden sein.

Aus der WO 2022/008481 A1 sind ein thermisches Desinfektionssystem und Verfahren zur Durchführung eines thermischen Desinfektionsverfahrens von Flüssigkeitsleitungen eines medizinischen Geräts bekannt. Das thermische Desinfektionsverfahren umfasst die Schritte: Empfangen eines Temperatursignals von einem Temperatursensor, Bestimmen eines gemessenen Temperaturwerts des Fluids innerhalb des Hydraulikkreislaufs, Empfangen eines Drucksignals von einem Drucksensor, Ermitteln eines gemessenen lokalen atmosphärischen Druckwerts und Ansteuern einer Heizeinheit zum Aufheizen des Fluids auf der Grundlage des gemessenen Temperaturwerts und des gemessenen lokalen atmosphärischen Druckwerts.

Der Erfindung liegt die Aufgabe zugrunde, ein verbessertes System und Verfahren zur Desinfektion einer Umkehrosmoseanlage bereitzustellen, welches die Nachteile aus dem Stand der Technik überwindet. Insbesondere soll die thermische Desinfektion der Umkehrosmoseanlage erfolgen können, ohne dass die beiden Anlagen miteinander signaltechnisch kommunizieren müssen.

In Bezug auf das System wird diese Aufgabe gelöst durch ein System mit den Merkmalen von Anspruch 1. Das System umfasst eine Umkehrosmoseanlage und eine Heißreinigungsanlage, die Umkehrosmoseanlage umfassend wenigstens eine Druckerhöhungspumpe und ein Membranmodul, wobei die Umkehrosmoseanlage über eine Zulaufleitung hydraulisch mit der Heißreinigungsanlage verbunden ist, und wobei eine Rücklaufleitung, in welche ein Magnetventil geschaltet ist, von der Heißreinigungsanlage zur Umkehrosmoseanlage führt, und wobei die Zulaufleitung hydraulisch mit der Rücklaufleitung durch eine Bypassleitung verbunden ist, in welche ein Bypassventil geschaltet ist, welches einen Fluss durch die Bypassleitung vollständig freigibt, wenn der Druck in der Zulaufleitung größer ist als ein Haltedruck des Bypassventils, wobei die Umkehrosmoseanlage eine Steuerungseinheit aufweist, welche folgende Schritte durchführt:
a) Öffnen des Magnetventils und Leiten von erhitztem Wasser aus der Heißreinigungsanlage durch die Rücklaufleitung in die Umkehrosmoseanlage;
b) Überprüfen des Desinfektionszustands der Umkehrosmoseanlage;
c) Sofern ein in der Steuerungseinheit hinterlegtes Desinfektionsziel der Umkehrosmoseanlage erreicht ist, Schließen des Magnetventils.

Vorteilhafte Ausgestaltungen der Erfindung sind Gegenstand der Unteransprüche.

Die Erfindung geht von der Überlegung aus, dass Desinfektionsziele der beiden Anlagen oder weiterer Komponenten, wie ein an einer Ringleitung angeschlossener Verbraucher, unterschiedlich definiert sein können und auch zu unterschiedlichen Zeitpunkten erreicht werden können.

Wie nunmehr erkannt wurde, lässt sich eine gleichzeitig vollständige und energiesparende Desinfektion der Umkehrosmoseanlage erreichen, indem die Umkehrosmoseanlage eigenständig das Erreichen ihres vorgegebenen Desinfektionsziels überwacht und bei Erreichen dieses Ziels die Förderung von Wasser in die Heißreinigungsanlage selbstständig stoppt.

Der Desinfektionszustand der Umkehrosmoseanlage wird vorteilhafterweise, bevorzugt iterativ, berechnet mit Hilfe von wiederholt an wenigstens einer Stelle in der Umkehrosmoseanlage gemessenen Temperaturen und der Zeitdauer der Heißreinigung. Bevorzugt wird die Temperatur an mehreren Stellen in der Umkehrosmoseanlage gemessen, insbesondere in regelmäßigen Abständen zwischen 100 ms und 300 ms, insbesondere alle 200 ms. Sofern die Temperatur nur an einer Stelle der Umkehrosmoseanlage gemessen wird, wird diese bevorzugt an der während des Verfahrens kältesten Stelle gemessen, sodass die erfolgreiche und vollständige Desinfektion der gesamten Umkehrosmoseanlage sichergestellt werden kann.

Vorteilhafterweise ist der Haltedruck des Bypassventils derart eingestellt, dass während des Schrittes a) durch die Bypassleitung nur ein Volumenstrom fließt, der kleiner oder gleich als ein vorgegebener Volumenstromschwellenwert ist. Auf diese Weise wird nur ein geringer Volumenstrom durch die Bypassleitung aufrechterhalten, während die Umkehrosmoseanlage mit Hilfe des erhitzten Wassers desinfiziert wird. Besonders bevorzugt wird kein (d.h. der Volumenstromschwellenwert ist Null) bzw. nur ein minimaler Volumenstrom während Phase a) über die Bypassleitung geführt und nur während Phase c) aktiv durchströmt.

Dies hat den Vorteil, dass entnommenes Wasser aus der Ringleitung über die hydraulische Verbindung direkt nachgeliefert wird, ohne dass dafür eine Kommunikation oder Aktion nötig wäre. Zudem wird keine überschüssige Energie benötigt, um die bereits desinfizierte Umkehrosmoseanlage weiter mit heißem Wasser zu versorgen.

In einer bevorzugten Ausführungsform ist an die Heißreinigungsanlage wenigstens eine Ringleitung mit wenigstens einem daran angeschlossenen Verbraucher hydraulisch angeschlossen, wobei erhitztes Wasser durch die Ringleitung gefördert wird und dem jeweiligen Verbraucher bereitgestellt wird. Auf diese Weise kann die thermische Desinfektion von Verbrauchern zumindest teilweise zeitgleich zur thermischen Desinfektion der Umkehrosmoseanlage erfolgen.

Das in der Steuerungseinheit der Umkehrosmoseanlage hinterlegte Desinfektionsziel der Umkehrosmoseanlage verwendet vorzugsweise einen A₀-Wert als Erfolgskontrolle gemäß DIN EN 15883. Der A₀-Wert ist dabei definiert durch die übliche Definition 1 A₀ = 1 Sekunde Einwirkzeit bei 80°C. Er dient als ein Maßstab für die Abtötung von Mikroorganismen in Verfahren mit feuchter Hitze. Bevorzugt ist für das Desinfektionsziel ein A₀-Wert vorgegeben, der im Fall der Desinfektion von Umkehrosmoseanlagen bei 600 oder höher liegt.

Die Berechnung des A₀-Wertes erfolgt iterativ durch Summation von Produkten aus dem jeweiligen Zeitschritt und einer Zehnerpotenz, bei welcher der Exponent gegeben ist durch die durch einen z-Wert geteilte Differenz der aktuell gemessenen Temperatur und 80° C. Der z-Wert ist dabei für den jeweiligen Mikroorganismus festgelegt und liegt im vorliegenden Fall bei 10°C.

Ein für Umkehrosmoseanlagen notwendiger A₀-Wert von mindestens 600 kann beispielsweise erreicht werden durch eine Desinfektion bei einer Temperatur von 80°C, welche 10 Minuten lang durchgeführt wird, oder bei einer Desinfektion von 100 min bei 70°C.

In einer ersten bevorzugten Ausführungsform ist das Bypassventil als Überströmventil ausgebildet. Der Haltedruck des Bypassventils bzw. des als Überströmventil ausgebildeten Bypassventils ist dabei derart konfiguriert, dass es einen Permeatfluss durch die Bypassleitung erlaubt, wenn der Druck in der Zulaufleitung zur Heißreinigung bzw. Heißreinigungsanlage größer ist als der Haltedruck des Überströmventils bzw. des als Überströmventil ausgebildeten Bypassventils. Auf diese Weise wird eine Zirkulation des Permeats ermöglicht, wenn nach Abschluss der thermischen Desinfektion bei Erreichen vorgegebenen Desinfektionsziels der Umkehrosmoseanlage das Magnetventil in der Rücklaufleitung geschlossen wird. Das Überströmventil weist bevorzugt eine Feder auf, durch welche der Haltedruck eingestellt wird. Die Feder ist dabei vorzugsweise derart ausgebildet, dass sie bei steigender Temperatur an Federkraft verliert. Dies sorgt für eine zusätzliche Reduzierung der Pumpendrehzahl der Druckerhöhungspumpe der Umkehrosmoseanlage.

In einer anderen bevorzugten Ausführungsform ist das Bypassventil als Magnetventil ausgebildet. Es ist signaltechnisch mit der Steuerungseinheit verbunden und während der thermischen Desinfektion der Umkehrosmose bzw. Umkehrosmoseanlage geschlossen, d.h. insbesondere die Steuerungseinheit schließt dieses Ventil. Es wird von ihr geöffnet, wenn nach Abschluss der thermischen Desinfektion das vorgegebene Desinfektionsziel der Umkehrosmoseanlage erreicht ist. Dies bedeutet, dass die Steuerungseinheit konfiguriert ist, insbesondere dass Programminstruktionen hardware- und/oder softwaremäßig in ihr hinterlegt sind, welche das beschriebene Öffnen und Schließen durchführen.

In einer bevorzugten Ausführungsform ist die Heißreinigungsanlage hydraulisch modular und elektrisch modular mit der Umkehrosmoseanlage verbunden und/oder verbindbar. Dies ermöglicht gewissermaßen einen Plug-und-Play-Betrieb der Heißreinigungsanlage. Diese ist vorteilhafterweise ein von der Umkehrosmoseanlage unabhängiges System und kann bedarfsweise mit der Umkehrosmoseanlage hydraulisch und elektrisch verbunden werden und wieder entfernt werden, wenn sie nicht mehr gebraucht wird. Sie wird dadurch einfach nachrüstbar und kann unabhängig von der Umkehrosmoseanlage gewartet und gereinigt werden.

Die Heißreinigungsanlage weist vorteilhafterweise eine Steuereinheit auf, wobei bei der elektrischen Verbindung der beiden Anlagen die Steuereinheit der Umkehrosmoseanlage und die Steuereinheit der Heißreinigungsanlage elektrisch isoliert voneinander sind. Bevorzugt wird die elektrische Isolierung dadurch erreicht, dass die sendende Steuerungseinheit ein Relais öffnen und schließen kann. Die empfangende Steuerungseinheit weist ein Signalleitungskabel auf, welches an die Relaiskontakte der sendenden Einheit angeschlossen ist. Wenn die sendende Einheit den Relaiskontakt wechselt, wird ein Zu- oder Abschalten an der Signalleitung erkannt, ohne dass die beiden Steuerungseinheiten bzw. elektrischen Kreise miteinander direkt in Verbindung stehen.

Für die hydraulische Verbindung wird bevorzugt ein identisches Anschlussmaß verwendet, wodurch das Herstellen und Lösen der hydraulischen Verbindung der beiden Anlagen miteinander vereinfacht wird.

Die Umkehrosmoseanlage und die Heißreinigungsanlage sind bevorzugt derart ausgebildet, dass über jeweils eine entsprechende Schnittstelle eine elektrische Verbindung der beiden Anlagen hergestellt werden kann, sodass dedizierte elektrische Signale von der Heißreinigungsanlage zur Steuereinheit der Umkehrosmoseanlage gesendet werden können. Da die Umkehrosmoseanlage ihr Desinfektionsziel selbst überwacht, ist hierbei bevorzugt nur eine Low-Level-Kommunikation vorgesehen (z.B. binäre Signale). Vorzugsweise wird dabei ermöglicht, dass die Heißreinigungsanlage bzw. an die Steuereinheit der Umkehrosmoseanlage ein Signal schickt, welches der Steuereinheit der Umkehrosmoseanlage anzeigt, dass nun die Heißreinigungsanlage verbunden ist. Ein weiteres Signal kann anzeigen, ob die Heißreinigungsanlage defekt oder verfügbar ist.

Bevorzugt sendet die Steuerung der Heißreinigungsanlage über ein Relais Signale an die Steuerung der Umkehrosmose. Umgekehrt sendet die Steuerung der Umkehrosmoseanlage über ein Relais Signale an die Steuerung der Heißreinigungsanlage.

Vorteilhafterweise werden dabei folgende Signale versendet:
RO-Bereit (VON Umkehrosmoseanlage AN Heißreinigungsanlage): Dieses Signal gibt der Heißreinigungsanlage an, dass diese die thermische Desinfektion starten kann und/oder eine thermische Desinfektion über die Steuerungseinheit (durch Nutzerinput oder automatisch nach Zeit) der Heißreinigung aktiviert werden kann.

Heißreinigungseinheit-Aktiv (VON Heißreinigungsanlage AN Umkehrosmoseanlage): Dieses Signal gibt der Umkehrosmose an, dass die Heißreinigung aktuell aktiv ist.

In Bezug auf das Verfahren wird die oben genannte Aufgabe erfindungsgemäß gelöst durch die Schritte des Vorgebens eines Desinfektionsziels der Um-kehrosmoseanlage, das Überprüfen des Desinfektionszustands der Umkehrosmoseanlage, und bei Erreichen des vorgegebenen Desinfektionsziels der Umkehrosmoseanlage das Beenden des Förderns von erhitztem Wasser durch die Rücklaufleitung und Fördern des erhitzten Wassers von der Zulaufleitung über eine Bypassleitung in die Rücklaufleitung.

Das Überprüfen des Desinfektionszustands der Umkehrosmoseanlage wird bevorzugt iterativ bzw. in regelmäßigen Abständen durchgeführt. Das Desinfektionsziel ist erreicht, wenn der Desinfektionszustand der Umkehrosmoseanlage dem definierten Desinfektionsziel entspricht.

Vorteilhafterweise wird während des Förderns von erhitztem Wasser durch die Rücklaufleitung durch die Bypassleitung nur ein Volumenstrom von erhitztem Wasser gefördert, der geringer als ein vorgegebener Volumenstromschwellenwert ist oder überhaupt kein Volumenstrom.

In einer bevorzugten Ausführungsform wird durch eine an die Heißreinigungsanlage angeschlossene Ringleitung erhitztes Wasser gefördert. Auf diese Weise können neben der Ringleitung auch Verbraucher, welche an Abnahmestellen an die Ringleitung angeschlossen sind, ebenfalls thermisch desinfiziert werden

Vorteilhafterweise wird wenigstens einem an der Ringleitung angeschlossenen Verbraucher erhitztes Wasser bereitgestellt, wobei die bereitgestellte Fördermenge an Flüssigkeit durch eine Druckerhöhungspumpe der Umkehrosmoseanlage nachgefördert wird.

Das Desinfektionsziel der Umkehrosmoseanlage ist vorzugsweise mit Hilfe des A₀-Wertes definiert.

Vorteilhafterweise wird durch eine Bypassleitung, welche die Zulaufleitung hydraulisch mit der Rücklaufleitung verbindet, Flüssigkeit gefördert, wenn der Druck in der Zulaufleitung größer ist als ein vorgegebener Haltedruck eines in die Bypassleitung geschalteten Bypassventils. Das Bypassventil ist dabei vorzugsweise als Überströmventil ausgebildet.

Die Vorteile der Erfindung liegen insbesondere darin, dass während der thermischen Desinfektion der Umkehrosmoseanlage die beiden Anlagen keine oder nur eine sehr rudimentäre Datenübertragung miteinander durchführen müssen. Es wird gewissermaßen eine Sparschaltung für die thermische Desinfektion einer Umkehrosmoseanlage bereitgestellt, da nur das Desinfektionsziel der Umkehrosmoseanlage berücksichtigt wird und nur bis zu dessen Erreichen die Umkehrosmoseanlage mit erhitztem Wasser gespült werden muss. Des Weiteren können, nachdem die Umkehrosmoseanlage desinfiziert wurde, weiterhin Verbraucher mit erhitztem Wasser versorgt werden. Dies ist energetisch von Vorteil, da der Wärmeverlust (über die Umkehrosmoseanlage) geringer ist und somit der Gesamtprozess effizienter wird. Es können unterschiedliche Desinfektionsziele von Ringleitung und Umkehrosmose realisiert werden.

Das Verfahren kann aber auch verwendet werden, wenn die beiden Anlagen miteinander verbunden sind und Daten austauschen. Dieser Datenaustausch kann verwendet werden um Statusmeldungen (Start der Heißreinigung, Erreichen des Desinfektionsziels etc.) bis hin zum Austauschen von Parametersätzen und Betriebsdaten.

Ein Ausführungsbeispiel der Erfindung wird nachfolgend anhand einer Zeichnung näher erläutert. Darin zeigen in stark schematisierter Darstellung:
- FIG. 1: ein System aus einer Umkehrosmoseanlage und einer Heißreinigungsanlage;
- FIG. 2: ein beispielhaftes Diagramm einer Pumpendrehzahl als Funktion der Zeit; und
- FIG. 3: ein Flussdiagramm eines Verfahrens zur thermischen Desinfektion einer Umkehrosmoseanlage in einer bevorzugten Ausführungsform.

Gleiche Teile sind in allen Figuren mit denselben Bezugszeichen versehen.

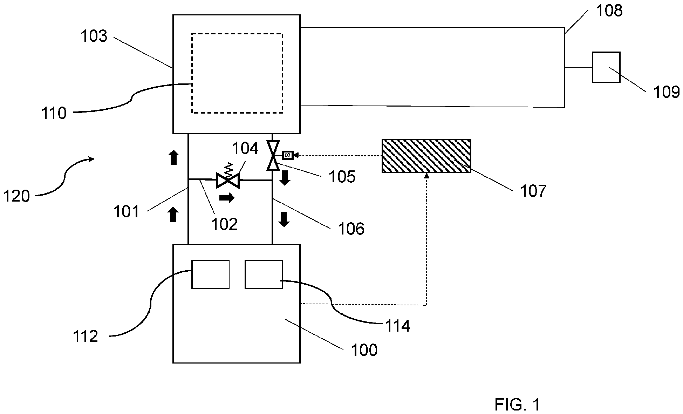

In FIG. 1 ist ein bevorzugtes Ausführungsbeispiel einer Umkehrosmoseanlage 100 und einer Heißreinigungsanlage 103 dargestellt. Die Umkehrosmoseanlage 100 ist über eine Zulaufleitung 101 mit der stromabwärts angeordneten Heißreinigungsanlage 103 hydraulisch verbunden. Umkehrosmoseanlage 100 und Heißreinigungsanlage 103 bilden ein System 120.

Von der Heißreinigungsanlage 103 führt eine hydraulische Rücklaufleitung 106 zur Umkehrosmoseanlage 100. In die Rücklaufleitung 106 ist ein Magnetventil 105 geschaltet. Die Heißreinigungsanlage 103 agiert in der Art eines Durchlauferhitzers, was in FIG. 1 durch eine gestrichelte Linie 110 dargestellt ist. Die Flüssigkeit, welche aus der Umkehrosmoseanlage 100 durch die Zulaufleitung 101 in die Heißreinigungsanlage 103 gefördert wird, wird in ihr erhitzt, bevor sie durch die Rücklaufleitung 106 in die Umkehrosmoseanlage 100 gefördert wird.

Die Umkehrosmoseanlage 100 weist eine Druckerhöhungspumpe 112 sowie wenigstens ein Membranmodul 114 auf, welches den zu filternden Flüssigkeitsstrom in bekannter Weise in Permeat und Konzentrat aufteilt.

Die Umkehrosmoseanlage 100 weist eine Steuerungseinheit 107 auf, in welcher ein gewünschtes bzw. vorgegebenes Desinfektionsziel hinterlegt ist. Das in der Steuerungseinheit 107 der Umkehrosmoseanlage hinterlegte Desinfektionsziel der Umkehrosmoseanlage verwendet vorzugsweise einen A₀-Wert als Erfolgskontrolle.

Die beiden Leitungen 101, 106 sind hydraulisch über eine Bypassleitung 102 miteinander verbunden, in welche ein Überströmventil 104 geschaltet ist. Durch die Bypassleitung 102 fließt nur Permeat, wenn der Druck in der Zulaufleitung 101 zur Heißreinigungsanlage 103 größer ist als der Haltedruck des Überströmventils 104. Dies kann zum Beispiel der Fall sein, wenn die Steuerungseinheit 107 das Magnetventil 105 in der Rücklaufleitung 106 zur Umkehrosmoseanlage 100 schließt.

Optional kann an der Heißreinigungsanlage 103 eine Ringleitung 108 mit einer Anzahl an Verbrauchern 109 (beispielsweise Dialysegeräte) angeschlossen sein. Diese Verbraucher 109 können heißes Wasser aus der Ringleitung 108 abnehmen, um sich selbst thermisch oder chemothermisch zu desinfizieren.

Die Heißreinigungsanlage 103 verfügt über eine eigene Pumpe, welche das Wasser in der Ringleitung 108 zirkuliert. Für die Dauer der aktiven Heißreinigung der Umkehrosmoseanlage 100 ist das Magnetventil 105 geöffnet. Der Haltedruck des Überströmventils 104 ist derart eingestellt, dass kein oder ein nur sehr geringer Volumenstrom über die Bypassleitung 102 fließt. Dadurch wird die Umkehrosmoseanlage 100 mit heißem Wasser von der Heißreinigungsanlage 103 über die Rücklaufleitung 106 versorgt. Sobald die Umkehrosmoseanlage 100 ihr Desinfektionsziel erreicht hat, schließt die Steuerungseinheit 107 das Magnetventil 105. Dadurch wird kein heißes Wasser zurück in die Umkehrosmoseanlage 100 geleitet. Somit kann die Heißreinigungsanlage 103 Heizenergie sparen, da die Umkehrosmoseanlage 100 kein heißes Wasser mehr verbraucht.

Sollte in der Zwischenzeit der wenigstens eine Verbraucher 109 Wasser aus der angeschlossenen Ringleitung 108 entnehmen, wird das Volumen automatisch über die Umkehrosmoseanlage 100 durch die Zulaufleitung 101 nachgefördert. Dies geschieht aufgrund der sich ändernden Druckverhältnisse aufgrund des fehlenden Volumens innerhalb der Heißreinigungsanlage 103 und der angeschlossenen Ringleitung 108. Somit kühlt sich ebenfalls über die Zeit die Umkehrosmoseanlage 100 ab und benötigt zu einem späteren Zeitpunkt eine geringere aktive Abkühlung. Dies liegt zum einen an der Abkopplung der Heißwasserversorgung von der Heißreinigungsanlage 103 und zum anderen am Ersetzen des durch den Verbraucher 109 abgenommenen Volumens durch kälteres Prozesseingangswasser/Weichwasser. Bevorzugt sind die hydraulischen Verbindungen zwischen der Umkehrosmoseanlage 100 (im speziellen bei den Leitungen 101, 106) identisch zu denen der Ringleitung 108. Dadurch wird das Nachrüsten der Heißreinigungseinheit bzw. Heißreinigungsanlage 103 bei bestehenden Systemen vereinfacht.

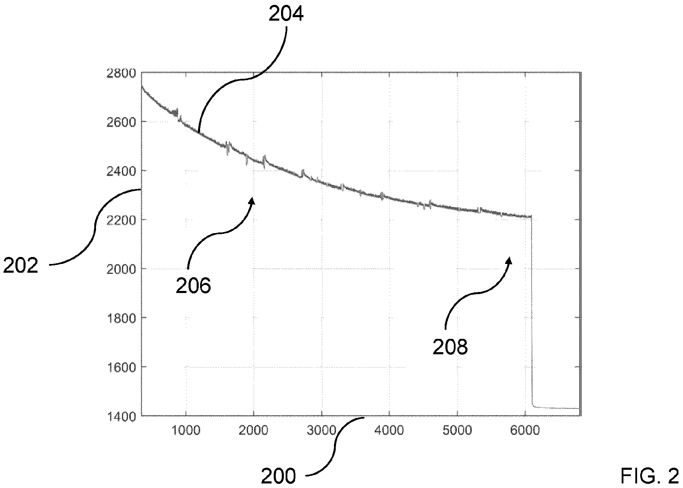

Besitzt die Umkehrosmoseanlage 100 geregelte Aktoren, wie beispielsweise Druckerhöhungspumpen und/oder Zirkulationspumpen, kann der Energieverbrauch ebenfalls gesenkt werden, da das heiße Wasser nicht mehr innerhalb der Umkehrosmoseanlage 100 zirkuliert werden muss und die Permeatproduktion nur noch das verbrauchte Volumen ersetzen muss. Dies sorgt für eine Verringerung der Pumpendrehzahl, wie dies beispielhaft in FIG. 2 dargestellt ist.

Bei dem in FIG. 2 dargestellten Diagramm ist auf einer x-Achse 200 die Zeit in Sekunden und auf einer y-Achse 202 die Pumpendrehzahl in Umdrehungen pro Minute einer geregelten Druckerhöhungspumpe 112 der Umkehrosmoseanlage 100 dargestellt. Eine Kurve 204 repräsentiert die Pumpendrehzahl. Die Pumpendrehzahl nimmt in einem ersten zeitlichen Abschnitt 206, während dessen erhitztes Wasser durch die Rücklaufleitung 106 in die Umkehrosmoseanlage 100 gefördert wird, im Lauf der Zeit langsam ab, bis sie zu Beginn eines zweiten zeitlichen Abschnitts 208, indem die Förderung von erhitztem Wasser in die Umkehrosmoseanlage 100 beendet wird, um ca. die Hälfte reduziert wird, um nur noch eine Grundzirkulation in der Umkehrosmoseanlage 100 aufrecht zu erhalten. Die zeitliche Abnahme der Pumpendrehzahl während der aktiven Heißreinigung im ersten zeitlichen Abschnitt 206 wird durch den sinkenden Widerstand des wenigstens einen Membranmoduls 114 hervorgerufen.

Besonders vorteilhaft bei dieser Ausgestaltung ist der abnehmende Haltedruck des Überströmventils 104 durch die steigende Temperatur. Dies liegt daran, dass der Haltedruck das Überströmventil 104 durch eine Feder eingestellt wird, welche bei steigender Temperatur an Federkraft verliert. Dies sorgt für eine zusätzliche Reduzierung der Pumpendrehzahl der Umkehrosmoseanlage 100.

Durch den dargestellten Aufbau und das beschriebene Verfahren ist keine Kommunikation zwischen den Geräten Umkehrosmoseanlage 100, Heißreinigungsanlage 103, Verbraucher 109 notwendig, um eine Heißreinigung durchzuführen. Ebenfalls können unterschiedliche Desinfektionsziele zwischen der Umkehrosmoseanlage 100 und den restlichen Systemkomponenten (103, 108, 109) realisiert werden, solange das Desinfektionsziel der Umkehrosmoseanlage 100 früher erreicht wird.

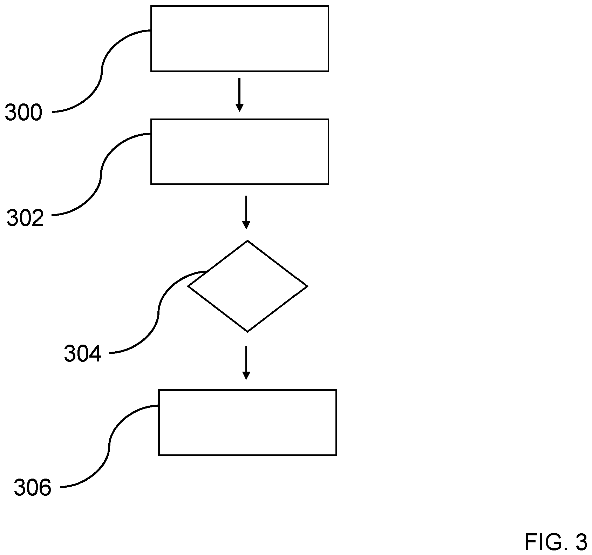

In FIG. 3 ist ein Verfahren zur thermischen Desinfektion einer Umkehrosmoseanlage 100 als Ablaufdiagramm dargestellt. In einem ersten Verfahrensschritt 300 wird ein Desinfektionsziel der Umkehrosmoseanlage 100 vorgegeben, welches vorliegend einen A₀-Wert als Erfolgskontrolle verwendet. In einem nachfolgenden Verfahrensschritt 302 wird aus der Umkehrosmoseanlage 100 Wasser durch eine Zulaufleitung 101 in eine Heißreinigungsanlage 103 gefördert und dort erhitzt. Das erhitzte Wasser aus der Heißreinigungsanlage 103 wird parallel durch eine Rücklaufleitung 106 in die Umkehrosmoseanlage 100 gefördert.

In einer Entscheidung 304 wird überprüft, ob das vorgegebene Desinfektionsziel bereits erreicht wurde. Ist dies nicht der Fall, verzweigt das Verfahren wieder zu Verfahrensschritt 302. Im anderen Fall wird ein Verfahrensschritt 306 ausgeführt, in welchem die Förderung von erhitztem Wasser durch die Rücklaufleitung 106 beendet wird und das erhitzte Wasser von der Zulaufleitung 101 über eine Bypassleitung 102 in die Rücklaufleitung 106 gefördert wird.

### Bezugszeichenliste

- 100: Umkehrosmoseanlage
- 101: Zulaufleitung
- 102: Bypassleitung
- 103: Heißreinigungsanlage
- 104: Überströmventil
- 105: Magnetventil
- 106: Rücklaufleitung
- 107: Steuerungseinheit
- 108: Ringleitung
- 109: Verbraucher
- 110: Linie
- 112: Druckerhöhungspumpe
- 114: Membranmodul
- 120: System
- 200: x-Achse
- 202: y-Achse
- 204: Kurve
- 206: erster zeitlicher Abschnitt
- 208: zweiter zeitlicher Abschnitt
- 300: Verfahrensschritt
- 302: Verfahrensschritt
- 304: Entscheidung
- 306: Verfahrensschritt

## Patentansprüche

1. System (120) aus Umkehrosmoseanlage (100) und Heißreinigungsanlage (103) zur thermischen Desinfektion der Umkehrosmoseanlage (100), die Um-kehrosmoseanlage (100) umfassend wenigstens eine Druckerhöhungspumpe (112) und ein Membranmodul (114),
wobei die Umkehrosmoseanlage (100) über eine Zulaufleitung (101) hydraulisch mit der Heißreinigungsanlage (103) verbunden ist, und wobei eine Rücklaufleitung (106), in welche ein Magnetventil (105) geschaltet ist, von der Heißreinigungsanlage (103) zur Umkehrosmoseanlage (100) führt, und wobei die Zulaufleitung (101) hydraulisch mit der Rücklaufleitung (106) durch eine Bypassleitung (102) verbunden ist, in welche ein Bypassventil (104) geschaltet ist, welches einen Fluss durch die Bypassleitung (102) vollständig freigibt, wenn der Druck in der Zulaufleitung (101) größer ist als ein Haltedruck des Bypassventils (104),
**dadurch gekennzeichnet, dass**
die Umkehrosmoseanlage (100) eine Steuerungseinheit (107) aufweist, welche folgende Schritte durchführt:
a) Öffnen des Magnetventils (105) und Leiten von erhitztem Wasser aus der Heißreinigungsanlage (103) durch die Rücklaufleitung (106) in die Umkehrosmoseanlage (100);
b) Überprüfen des Desinfektionszustands der Umkehrosmoseanlage (100);
c) Sofern ein in der Steuerungseinheit (107) hinterlegtes Desinfektionsziel der Umkehrosmoseanlage (100) erreicht ist, Schließen des Magnetventils (105).

2. System (120) nach Anspruch 1, wobei der Haltedruck des Bypassventils (104) derart eingestellt ist, dass während des Schrittes a) durch die Bypassleitung (102) nur ein Volumenstrom fließt, der kleiner oder gleich als ein vorgegebener Volumenstromschwellenwert ist.

3. System (120) nach Anspruch 1 oder 2, wobei an die Heißreinigungsanlage (103) eine Ringleitung (108) mit wenigstens einem daran angeschlossenen Verbraucher (109) hydraulisch angeschlossen ist, und wobei heißes Wasser durch die Ringleitung (108) gefördert wird und dem jeweiligen Verbraucher (109) bereitgestellt wird.

4. System (120) nach einem der Ansprüche 1 bis 3, wobei das in der Steuerungseinheit (107) hinterlegte Desinfektionsziel der Umkehrosmoseanlage (100) einen A₀-Wert als Erfolgskontrolle verwendet.

5. System (120) nach einem der Ansprüche 1 bis 4, wobei das Bypassventil (104) als Überströmventil ausgebildet ist.

6. System (120) nach Anspruch 5, wobei der Haltedruck des als Überströmventil ausgebildeten Bypassventils (104) derart konfiguriert ist, dass es einen Permeatfluss durch die Bypassleitung (102) erlaubt, wenn der Druck in der Zulaufleitung (101) größer ist als der Haltedruck des als Überströmventil ausgebildeten Bypassventils (104).

7. System (120) nach einem der Ansprüche 1 bis 4, wobei das Bypassventil (104) als Magnetventil ausgebildet ist.

8. System (120) nach Anspruch 7, wobei das als Magnetventil ausgebildete Bypassventil (104) signaltechnisch mit der Steuerungseinheit (107) verbunden und während der thermischen Desinfektion der Umkehrosmoseanlage (100) von der Steuereinheit (107) geschlossen wird, wobei das als Magnetventil ausgebildete Bypassventil (104) von der Steuereinheit (107) geöffnet wird, wenn nach Abschluss der thermischen Desinfektion das vorgegebene Desinfektionsziel der Umkehrosmoseanlage (100) erreicht ist.

9. System (120) nach einem der vorherigen Ansprüche, wobei die Heißreinigungsanlage (103) hydraulisch modular und elektrisch modular mit der Umkehrosmoseanlage (100) verbunden und/oder verbindbar ist.

10. System (120) nach Anspruch 9, wobei die Heißreinigungsanlage (103) eine Steuereinheit aufweist, und wobei bei der elektrischen Verbindung der beiden Anlagen (100, 103) die Steuereinheit (107) der Umkehrosmoseanlage und die Steuereinheit der Heißreinigungsanlage (103) elektrisch isoliert voneinander sind.

11. Verfahren zur thermischen Desinfektion einer Umkehrosmoseanlage (100), wobei aus der Umkehrosmoseanlage (100) Wasser durch eine Zulaufleitung (101) in eine Heißreinigungsanlage (103) gefördert und dort erhitzt wird, und wobei das erhitzte Wasser aus der Heißreinigungsanlage (103) durch eine Rücklaufleitung (106) in die Umkehrosmoseanlage (100) gefördert wird,
**gekennzeichnet durch** die Schritte
• Vorgeben eines Desinfektionsziel der Umkehrosmoseanlage (100);
• Überprüfen des Desinfektionszustands der Umkehrosmoseanlage (100);
• bei Erreichen des vorgegebenen Desinfektionsziels der Umkehrosmoseanlage (100), Beenden der Förderung von erhitztem Wasser durch die Rücklaufleitung (106) und Fördern des erhitzten Wassers von der Zulaufleitung (106) über eine Bypassleitung (102) in die Rücklaufleitung (101).

12. Verfahren nach Anspruch 11, wobei während des Förderns von erhitztem Wasser durch die Rücklaufleitung (106) durch die Bypassleitung (102) nur ein Volumenstrom von erhitztem Wasser gefördert wird, der geringer als oder gleich einem vorgegebener Volumenstromschwellenwert ist.

13. Verfahren nach Anspruch 11 oder 12, wobei durch eine an die Heißreinigungsanlage (103) angeschlossene Ringleitung (108) erhitztes Wasser gefördert wird.

14. Verfahren nach Anspruch 13, wobei wenigstens einem an der Ringleitung (108) angeschlossenen Verbraucher (109) erhitztes Wasser bereitgestellt wird, und wobei die bereitgestellte Fördermenge durch eine Druckerhöhungspumpe der Umkehrosmoseanlage (100) nachgefördert wird.

15. Verfahren nach einem der Ansprüche 11 bis 14, wobei durch eine Bypassleitung (102), welche die Zulaufleitung (101) hydraulisch mit der Rücklaufleitung (106) verbindet, Flüssigkeit gefördert wird, wenn der Druck in der Zulaufleitung (101) größer ist als ein vorgegebener Haltedruck eines in die Bypassleitung (102) geschalteten Bypassventils (104).
